**Europäisches Patentamt**

**European Patent Office**

**Office européen des brevets**

(11) Numéro de publication : **0 350 438 B1**

(12)
# FASCICULE DE BREVET EUROPEEN

(45) Date de publication du fascicule du brevet :
**09.09.92 Bulletin 92/37**

(51) Int. Cl.$^5$ : **A61K 7/48,** A61K 31/07

(21) Numéro de dépôt : **89810435.1**

(22) Date de dépôt : **07.06.89**

(54) Composition à usage cosmétique ou pharmaceutique.

(30) Priorité : **13.06.88 CH 2259/88**
**03.10.88 CH 3661/88**

(43) Date de publication de la demande :
**10.01.90 Bulletin 90/02**

(45) Mention de la délivrance du brevet :
**09.09.92 Bulletin 92/37**

(84) Etats contractants désignés :
**AT BE DE ES FR GB GR IT LU NL SE**

(56) Documents cités :
**FR-A- 2 378 523**

(73) Titulaire : **HERZOG, Paul**
**Route du Monteliza**
**CH-1806 St-Légier (CH)**
Titulaire : **HERZOG- THOMANDER, Karin**
**Route du Monteliza**
**CH-1806 St-Légier (CH)**

(72) Inventeur : **Herzog, Paul**
**Route de Monteliza**
**CH-1806 Saint-Légier (CH)**

(74) Mandataire : **Vuille, Roman**
**c/o Debiopharm S.A. 15-17, Rue des Terreaux**
**Case Postale 82**
**CH-1003 Lausanne (CH)**

## Description

La présente invention a pour objet une nouvelle composition à usage cosmétique ou pharmaceutique. Essentiellement destinée à un usage externe, elle permet de traiter efficacement diverses affections de la peau.

L'action thérapeutique de la vitamine A sous sa forme acide (acide rétinoïque), aldéhyde (rétinal) ou alcool (rétinol) est bien connue en dermatologie. Cette action est notamment mise à profit dans le traitement des proliférations cutanées, de l'acné, du psoriasis ou de troubles analogues, ainsi que pour la stimulation de la cicatrisation des blessures par exemple. L'administration du principe actif s'effectue par voie interne aussi bien que par voie externe.

Pour ce qui est du traitement topique d'affections telles que l'acné, on a également proposé d'utiliser conjointement l'acide rétinoïque (vitamine A-acide) et le peroxyde de benzoyle, un produit oxydant connu. Lors de tels traitements, on est cependant confronté à des effets secondaires désagréables sinon néfastes, notamment dus à la formation d'acide benzoïque suite à la décomposition du peroxyde correspondant: irritation, desquamation et même lésions de muqueuses dans certains cas. De tels effets secondaires ne permettent pas d'envisager sans risques l'application cosmétique de telles préparations.

Pour l'heure, on ne dispose pas de compositions à usage cosmétique aussi bien que pharmaceutique associant les effets de la vitamine A et d'un produit oxydant du type peroxyde, mais qui ne présente pas d'effets secondaires indésirables. Grâce à la nouvelle composition, selon l'invention, on peut aisément surmonter ces inconvénients.

L'invention a plus précisément pour objet une composition à usage cosmétique ou pharmaceutique, caractérisée en ce qu'elle contient à titre d'ingrédient actif de la vitamine A sous forme acide ou un ester de vitamine A, en combinaison avec une émulsion aqueuse de peroxyde d'hydrogène.

On utilise de préférence un ester de vitamine A, tel un ester d'acide gras comme le palmitate de vitamine A par exemple, ou encore l'acétate correspondant. De tels esters ainsi que la forme acide de la vitamine A se trouvent dans le commerce sous une forme appropriée; les esters d'acide gras de vitamine A ont en outre l'avantage d'être très bien tolérés par la peau et l'organisme en général, car certains d'entre eux sont des métabolites naturels du rétinol (forme alcool de la vitamine A).

La forme acide de la vitamine A ainsi que les esters susmentionnés s'utilisent, conformément à l'invention, en association avec une émulsion aqueuse stable de peroxyde d'hydrogène, les effets respectifs de ces deux composants se combinant de façon particulièrement efficace.

Etant donné l'absence de produits secondaires toxiques lors de la décomposition du peroxyde d'hydrogène (eau oxygénée), de telles émulsions peuvent présenter une concentration élevée en oxygène naissant qui renforce d'autant l'action de la vitamine A ou de son ester au niveau de l'épiderme.

Des émulsions stables utilisables dans le cadre de la présente invention peuvent être du type eau-dans-huile aussi bien que huile-dans-eau. Une émulsion huile-dans-eau stable, particulièrement bien adaptée à la préparation de compositions selon l'invention, est décrite dans le brevet US 3.954.874.

Une telle émulsion possède un pouvoir libérateur d'oxygène particulièrement élevé (pression paraosmotique de près de 10 V 1013, 25 kPa (atm)), ce qui a pour effet d'accélérer le passage de la vitamine A ou de son ester au travers des couches superficielles de la peau. Pénétrant dans une zone particulièrement riche en oxygène (augmentation de l'activité des vaisseaux capillaires stimulée par l'oxygène libéré), l'ester de vitamine A est rapidement métabolisé en rétinol, qui s'insère ensuite dans la chaîne métabolique cellulaire. On a ainsi pu constater une certaine stimulation de la croissance cellulaire de même qu'une augmentation de la formation de collagène.

De ce fait, les compositions selon l'invention conviennent parfaitement bien à de nombreux usages cosmétiques et, en particulier, à la lutte contre le vieillissement précoce de la peau. Les concentrations relatives en vitamine A (forme acide) ou en ester de vitamine A peuvent varier fortement, en fonction des effets recherchés. Pour des applications de type pharmaceutique, on peut envisager selon les cas des concentrations supérieures à celle figurant dans les exemples ci-dessous. Il en va de même pour ce qui est de la concentration initiale en oxygène actif, c'est-à-dire du pouvoir oxygénant cherché.

Les compositions selon l'invention peuvent bien entendu contenir les ingrédients stabilisants, épaississants, parfumants ou colorants usuels. Elles peuvent également contenir des ingrédients actifs additionnels, par exemple d'autres vitamines ou dérivés de vitamines telle la vitamine E ou des liposomes par exemple.

Etant à base d'émulsion aqueuse à pouvoir oxygénant élevé, les compositions selon l'invention présentent en outre un effet désinfectant et cicatrisant fort avantageux et s'avèrent utiles pour le traitement de brûlures, de blessures ouvertes tels les ulcères ou les troubles consécutifs aux hémoroïdes.

Les exemples ci-après sont destinés à illustrer l'invention de manière plus détaillée.

EXEMPLE 1

Composition cosmétique

On prépare une première phase de type "huile" en mélangeant les ingrédients suivants:

| | |
|---|---|
| Vaseline | 450 g |
| Paraffine liquide | 325 g |
| Alcool cétylique | 160 g |
| Alcool stéarylique | 160 g |
| Monostéarine | 310 g |
| Total | 1405 g |

On effectue séparément le mélange des constituants suivants pour obtenir une phase de type "eau":

| | |
|---|---|
| $H_2O_2$ 30% | 400 g |
| "Tween 80" | 125 g |
| Acide salicylique | 9 g |
| Palmitate de vitamine A[*] | 63 g |
| Acétate de D,L-$\alpha$-Tocophérol | 200 g |
| Eau distillée | 7798 g |
| Total | 8598 g |

\* 1,7 mio U.I./g

Les phases "huile" et "eau" ainsi préparées sont ensuite mélangées à température de 70 à 80°C dans un appareillage adéquat, jusqu'à obtention d'une émulsion homogène. On obtient de la sorte 10 kg d'une crème relativement consistante, à usage cosmétique.

A noter que dans ce cas, la quantité d'acétate d'$\alpha$-tocophérol introduite (acétate de vitamine E/antioxydant) est supérieure à la quantité nécessaire à sa fonction purement antioxydante. Il s'agit donc d'une composition enrichie en vitamine E.

EXEMPLE 2

Composition cosmétique

On prépare une première phase de type "huile" en mélangeant les ingrédients suivants:

| | |
|---|---|
| Vaseline | 450 g |
| Paraffine liquide | 325 g |
| Alcool cétylique | 160 g |
| Alcool stéarylique | 160 g |
| Monostéarine | 310 g |
| Total | 1405 g |

On effectue séparément le mélange des constituants suivants pour obtenir une phase de type "eau":

| | |
|---|---|
| $H_2O_2$ 30% | 400 g |
| "Tween 80" | 125 g |
| Acide salicylique | 9 g |
| Acétate de vitamine A* | 37. g |
| Acétate de D,L-α-Tocophérol | 200 g |
| Eau distillée | 7824 g |
| Total | 8595 g |

\* 2,9 mio U.I./g

Les phases "eau" et "huile" ci-dessus ont ensuite été traitées comme indiqué à l'Exemple 1.

EXEMPLE 3

Composition cosmétique

On prépare une première phase de type "huile" en mélangeant les ingrédients suivants:

| | |
|---|---|
| Vaseline | 450 g |
| Paraffine liquide | 325 g |
| Alcool cétylique | 160 g |
| Alcool stéarylique | 160 g |
| Monostéarine | 310 g |
| Total | 1405 g |

On effectue séparément le mélange des constituants suivants pour obtenir une phase de type "eau":

4

| | |
|---|---|
| $H_2O_2$ 30% | 400 g |
| "Tween 80" | 125 g |
| Acide salicylique | 9 g |
| Acide rétinoïque* | 5 g |
| Acétate de D,L-$\alpha$-Tocophérol | 200 g |
| Eau distillée | 7856 g |
| Total | 8595 g |

\* Vitamine A sous forme acide (pureté 100%)

Les phases "eau" et "huile" ci-dessus ont ensuite été traitées comme indiqué à l'Exemple 1.

Ces compositions se sont avérées particulièrement efficaces dans les applications cosmétiques visant à lutter contre le vieillissement de la peau. Elles peuvent être également utilisées dans le traitement d'affections bénignes de la peau telles que l'acné. Ces compositions possèdent en outre un effet désinfectant.

## Revendications

1. Composition à usage cosmétique ou pharmaceutique, caractérisée en ce qu'elle contient à titre d'ingrédient actif de la vitamine A sous forme acide ou un ester de vitamine A,en combinaison avec une émulsion aqueuse de peroxyde d'hydrogène.

2. Composition selon la revendication 1, caractérisée en ce que l'ester de vitamine A est choisi parmi le palmitate et l'acétate de vitamine A.

3. Composition selon la revendication 1 ou 2, caractérisée en ce que l'émulsion aqueuse de peroxyde d'hydrogène est une émulsion huile-dans-eau.

## Patentansprüche

1. Mittel zur kosmetischen oder pharmazeutischen Verwendung, dadurch gekennzeichnet, dass es Vitamin A Saüre oder ein Vitamin A Ester mit einer wässrigen Wasserstoffperoxyd Emulsion kombiniert, als aktiver Bestanteil enthält.

2. Mittel gemäss Anspruch 1, dadurch gekennzeichnet, dass das Vitamin A Ester Vitamin A Palmitat oder Vitamin A Essigsaüreester ist.

3. Mittel gemäss Anspruch 1 oder 2, dadurch gekennzeichnet, die wässrige Wasserstoffperoxyd Emulsion eine Oel-in-Wasser Emulsion ist.

## Claims

1. Composition for cosmetic or pharmaceutical use comprising as active ingredient Vitamin A as an acid or a Vitamin A ester combined with an aqueous hydrogenperoxide emulsion.

2. Composition according to claim 1, wherein the ester of Vitamin A is selected from Vitamin A palmitate or Vitamin A acetate.

3. Composition according to claim 1 or 2, wherein the aqueous hydrogenperoxide emulsion is an oil-in-water emulsion.